# EUROPEAN PATENT APPLICATION

(11) **EP 3 967 706 A1**
(43) Date of publication of application: **16.03.2022**
(21) Application number: 19927618.9
(22) Date of filing: 08.05.2019
(51) Int. Cl.: C07K 14/075, C12N 7/01, C12N 15/861, A61K 48/00, A61P 27/16

(54) **AAV MUTANT THAT EFFICIENTLY INFECTS SUPPORTING CELLS**

(71) Applicant: Center for Excellence in Brain Science and Intelligence Technology, Chinese Academy of Sciences, Xuhui District Shanghai 200031 (CN)
(72) Inventor: YANG, Hui, Shanghai 200031 (CN); YAO, Xuan, Shanghai 200031 (CN); WANG, Xing, Shanghai 200031 (CN); SHI, Linyu, Shanghai 200031 (CN)
(74) Representative: Cabinet Laurent & Charras
(86) International application number: PCT/CN2019/086050
(87) International publication number: WO 2020/223933

(57) **Abstract**

Provided is an AAV mutant that efficiently infects supporting cells. Specifically, provided is a gene expression vector used for treating hearing disorders, and the described expression vector is an AAV mutant. In the AAV mutant, an expression cassette of a therapeutic gene used for treating hearing disorders is inserted or carried, and additionally, the AAV mutant contains a gene sequence that encodes a viral capsid protein mutant.

sequence listing

<110>  CENTER FOR EXCELLENCE IN BRAIN SCIENCE AND INTELLIGENCE TECHNOLOGY,
CHINESE ACADEMY OF SCIENCES

<120>  AAV MUTANT THAT EFFICIENTLY INFECTS SUPPORTING CELLS

<130>  X7-B-63915 EP

<140>  PCT/CN2019/086050
<141>  2019-05-08

<160>  4

<170>  PatentIn version 3.5

<210>  1
<211>  737
<212>  PRT
<213>  artificial sequence

<220>
<223> the wild-type viral capsid protein

<400>  1

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu His Ser Pro Val Glu Pro Asp Ser Ser Ser Gly Thr Gly

145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Ile Gly Glu Pro Pro
            180                 185                 190

Ala Ala Pro Ser Gly Val Gly Ser Leu Thr Met Ala Ala Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Arg Leu Ser Phe Lys Leu Phe Asn Ile
305                 310                 315                 320

Gln Val Lys Glu Val Thr Gln Asn Glu Gly Thr Lys Thr Ile Ala Asn
                325                 330                 335

Asn Leu Thr Ser Thr Ile Gln Val Phe Thr Asp Ser Glu Tyr Gln Leu
            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Thr Tyr Thr
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Arg Thr Gln Thr Thr Gly Gly Thr Thr Asn Thr Gln Thr Leu Gly Phe
    450                 455                 460

Ser Gln Gly Gly Pro Asn Thr Met Ala Asn Gln Ala Lys Asn Trp Leu
465                 470                 475                 480

Pro Gly Pro Cys Tyr Arg Gln Gln Arg Val Ser Lys Thr Ser Ala Asp
                485                 490                 495

Asn Asn Asn Ser Glu Tyr Ser Trp Thr Gly Ala Thr Lys Tyr His Leu
            500                 505                 510

Asn Gly Arg Asp Ser Leu Val Asn Pro Gly Pro Ala Met Ala Ser His
        515                 520                 525

Lys Asp Asp Glu Glu Lys Phe Phe Pro Gln Ser Gly Val Leu Ile Phe
    530                 535                 540

Gly Lys Gln Gly Ser Glu Lys Thr Asn Val Asp Ile Glu Lys Val Met
545                 550                 555                 560

Ile Thr Asp Glu Glu Glu Ile Arg Thr Thr Asn Pro Val Ala Thr Glu
                565                 570                 575

Gln Tyr Gly Ser Val Ser Thr Asn Leu Gln Arg Gly Asn Arg Gln Ala
            580                 585                 590

Ala Thr Ala Asp Val Asn Thr Gln Gly Val Leu Pro Gly Met Val Trp
        595                 600                 605

Gln Asp Arg Asp Val Tyr Leu Gln Gly Pro Ile Trp Ala Lys Ile Pro
    610                 615                 620

His Thr Asp Gly His Phe His Pro Ser Pro Leu Met Gly Gly Phe Gly
625                 630                 635                 640

Leu Lys His Pro Pro Pro Gln Ile Leu Ile Lys Asn Thr Pro Val Pro
                645                 650                 655

Ala Asp Pro Pro Thr Thr Phe Asn Gln Ser Lys Leu Asn Ser Phe Ile
            660                 665                 670

Thr Gln Tyr Ser Thr Gly Gln Val Ser Val Glu Ile Glu Trp Glu Leu
        675                 680                 685

Gln Lys Glu Asn Ser Lys Arg Trp Asn Pro Glu Ile Gln Tyr Thr Ser
    690                 695                 700

Asn Tyr Tyr Lys Ser Thr Ser Val Asp Phe Ala Val Asn Thr Glu Gly
705                 710                 715                 720

Val Tyr Ser Glu Pro Arg Pro Ile Gly Thr Arg Tyr Leu Thr Arg Asn
                725                 730                 735

Leu

<210>  2
<211>  737
<212>  PRT
<213>  artificial sequence

<220>
<223> the viral capsid protein mutant

<400>  2

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Ile Gly Glu Pro Pro
            180                 185                 190

Ala Ala Pro Ser Gly Val Gly Ser Leu Thr Met Ala Ala Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Arg Leu Ser Phe Lys Leu Phe Asn Ile
305                 310                 315                 320

Gln Val Lys Glu Val Thr Gln Asn Glu Gly Thr Lys Thr Ile Ala Asn
                325                 330                 335

Asn Leu Thr Ser Thr Ile Gln Val Phe Thr Asp Ser Glu Tyr Gln Leu
            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Thr Tyr Thr
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Arg Thr Gln Thr Thr Gly Gly Thr Thr Asn Thr Gln Thr Leu Gly Phe
    450                 455                 460

Ser Gln Gly Gly Pro Asn Thr Met Ala Asn Gln Ala Lys Asn Trp Leu
465                 470                 475                 480

Pro Gly Pro Cys Tyr Arg Gln Gln Arg Val Ser Lys Thr Ser Ala Asp
                485                 490                 495

Asn Asn Asn Ser Glu Tyr Ser Trp Thr Gly Ala Thr Lys Tyr His Leu
            500                 505                 510

Asn Gly Arg Asp Ser Leu Val Asn Pro Gly Pro Ala Met Ala Ser His
        515                 520                 525

Lys Asp Asp Glu Glu Lys Phe Phe Pro Gln Ser Gly Val Leu Ile Phe
    530                 535                 540

Gly Lys Gln Gly Ser Glu Lys Thr Asn Val Asp Ile Glu Lys Val Met
545                 550                 555                 560

Ile Thr Asp Glu Glu Glu Ile Arg Thr Thr Asn Pro Val Ala Thr Glu
                565                 570                 575

Gln Tyr Gly Ser Val Ser Thr Asn Leu Gln Arg Gly Asn Arg Gln Ala
            580                 585                 590

Ala Thr Ala Asp Val Asn Thr Gln Gly Val Leu Pro Gly Met Val Trp
        595                 600                 605

Gln Asp Arg Asp Val Tyr Leu Gln Gly Pro Ile Trp Ala Lys Ile Pro
    610                 615                 620

His Thr Asp Gly His Phe His Pro Ser Pro Leu Met Gly Gly Phe Gly
625                 630                 635                 640

Leu Lys His Pro Pro Pro Gln Ile Leu Ile Lys Asn Thr Pro Val Pro
                645                 650                 655

Ala Asp Pro Pro Thr Thr Phe Asn Gln Ser Lys Leu Asn Ala Phe Ile
            660                 665                 670

Thr Gln Tyr Ser Thr Gly Gln Val Ser Val Glu Ile Glu Trp Glu Leu
        675                 680                 685

Gln Lys Glu Asn Ser Lys Arg Trp Asn Pro Glu Ile Gln Tyr Thr Ser
    690                 695                 700

Asn Tyr Tyr Lys Ser Thr Ser Val Asp Phe Ala Val Asn Thr Glu Gly
705                 710                 715                 720

Val Tyr Ser Glu Pro Arg Pro Ile Gly Thr Arg Tyr Leu Thr Arg Asn
                725                 730                 735

Leu

<210>  3
<211>  737
<212>  PRT
<213>  artificial sequence

<220>
<223> the viral capsid protein mutant

<400>  3

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Ile Gly Glu Pro Pro
            180                 185                 190

Ala Ala Pro Ser Gly Val Gly Ser Leu Thr Met Ala Ala Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Ala Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Arg Leu Ser Phe Lys Leu Phe Asn Ile
305                 310                 315                 320

Gln Val Lys Glu Val Thr Gln Asn Glu Gly Thr Lys Thr Ile Ala Asn
                325                 330                 335

Asn Leu Thr Ser Thr Ile Gln Val Phe Thr Asp Ser Glu Tyr Gln Leu
            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Thr Tyr Thr
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Arg Thr Gln Thr Thr Gly Gly Thr Thr Asn Thr Gln Thr Leu Gly Phe
    450                 455                 460

Ser Gln Gly Gly Pro Asn Thr Met Ala Asn Gln Ala Lys Asn Trp Leu
465                 470                 475                 480

Pro Gly Pro Cys Tyr Arg Gln Gln Arg Val Ser Lys Thr Ser Ala Asp
                485                 490                 495

Asn Asn Asn Ser Glu Tyr Ser Trp Thr Gly Ala Thr Lys Tyr His Leu
            500                 505                 510

Asn Gly Arg Asp Ser Leu Val Asn Pro Gly Pro Ala Met Ala Ser His
        515                 520                 525

Lys Asp Asp Glu Glu Lys Phe Phe Pro Gln Ser Gly Val Leu Ile Phe
    530                 535                 540

Gly Lys Gln Gly Ser Glu Lys Thr Asn Val Asp Ile Glu Lys Val Met
545                 550                 555                 560

Ile Thr Asp Glu Glu Glu Ile Arg Thr Thr Asn Pro Val Ala Thr Glu
                565                 570                 575

Gln Tyr Gly Ser Val Ser Thr Asn Leu Gln Arg Gly Asn Arg Gln Ala

580                 585                 590

Ala Thr Ala Asp Val Asn Thr Gln Gly Val Leu Pro Gly Met Val Trp
        595                 600                 605

Gln Asp Arg Asp Val Tyr Leu Gln Gly Pro Ile Trp Ala Lys Ile Pro
    610                 615                 620

His Thr Asp Gly His Phe His Pro Ser Pro Leu Met Gly Gly Phe Gly
625                 630                 635                 640

Leu Lys His Pro Pro Pro Gln Ile Leu Ile Lys Asn Thr Pro Val Pro
                645                 650                 655

Ala Asp Pro Pro Thr Thr Phe Asn Gln Ser Lys Leu Asn Ser Phe Ile
            660                 665                 670

Thr Gln Tyr Ser Thr Gly Gln Val Ser Val Glu Ile Glu Trp Glu Leu
        675                 680                 685

Gln Lys Glu Asn Ser Lys Arg Trp Asn Pro Glu Ile Gln Tyr Thr Ser
    690                 695                 700

Asn Tyr Tyr Lys Ser Thr Ser Val Asp Phe Ala Val Asn Thr Glu Gly
705                 710                 715                 720

Val Tyr Ser Glu Pro Arg Pro Ile Gly Thr Arg Tyr Leu Thr Arg Asn
                725                 730                 735

Leu

<210>  4
<211>  737
<212>  PRT
<213>  artificial sequence

<220>
<223> the viral capsid protein mutant

<400>  4

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Ile Gly Glu Pro Pro
            180                 185                 190

Ala Ala Pro Ser Gly Val Gly Ser Leu Thr Met Ala Ala Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn

290                 295                 300

Asn Trp Gly Phe Arg Pro Lys Arg Leu Ser Phe Lys Leu Phe Asn Ile
305                 310                 315                 320

Gln Val Lys Glu Val Thr Gln Asn Glu Gly Thr Lys Thr Ile Ala Asn
                325                 330                 335

Asn Leu Thr Ser Thr Ile Gln Val Phe Thr Asp Ser Glu Tyr Gln Leu
            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Thr Tyr Thr
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Arg Thr Gln Thr Thr Gly Gly Thr Thr Asn Thr Gln Thr Leu Gly Phe
    450                 455                 460

Ser Gln Gly Gly Pro Asn Thr Met Ala Asn Gln Ala Lys Asn Trp Leu
465                 470                 475                 480

Pro Gly Pro Cys Tyr Arg Gln Gln Arg Val Ser Lys Thr Ser Ala Asp
                485                 490                 495

Asn Asn Asn Ser Glu Tyr Ser Trp Thr Gly Ala Thr Lys Tyr His Leu
            500                 505                 510

Asn Gly Arg Asp Ser Leu Val Asn Pro Gly Pro Ala Met Ala Ser His
        515                 520                 525

Lys Asp Asp Glu Glu Arg Phe Phe Pro Gln Ser Gly Val Leu Ile Phe
    530                 535                 540

Gly Lys Gln Gly Ser Glu Lys Thr Asn Val Asp Ile Glu Lys Val Met
545                 550                 555                 560

Ile Thr Asp Glu Glu Glu Ile Arg Thr Thr Asn Pro Val Ala Thr Glu
                565                 570                 575

Gln Tyr Gly Ser Val Ser Thr Asn Leu Gln Arg Gly Asn Arg Gln Ala
            580                 585                 590

Ala Thr Ala Asp Val Asn Thr Gln Gly Val Leu Pro Gly Met Val Trp
        595                 600                 605

Gln Asp Arg Asp Val Tyr Leu Gln Gly Pro Ile Trp Ala Lys Ile Pro
    610                 615                 620

His Thr Asp Gly His Phe His Pro Ser Pro Leu Met Gly Gly Phe Gly
625                 630                 635                 640

Leu Lys His Pro Pro Pro Gln Ile Leu Ile Lys Asn Thr Pro Val Pro
                645                 650                 655

Ala Asp Pro Pro Thr Thr Phe Asn Gln Ser Lys Leu Asn Ser Phe Ile
            660                 665                 670

Thr Gln Tyr Ser Thr Gly Gln Val Ser Val Glu Ile Glu Trp Glu Leu
        675                 680                 685

Gln Lys Glu Asn Ser Lys Arg Trp Asn Pro Glu Ile Gln Tyr Thr Ser
    690                 695                 700

Asn Tyr Tyr Lys Ser Thr Ser Val Asp Phe Ala Val Asn Thr Glu Gly
705                 710                 715                 720

Val Tyr Ser Glu Pro Arg Pro Ile Gly Thr Arg Tyr Leu Thr Arg Asn
                725                 730                 735

Leu

## Description

### Technical field

The present invention belongs to the field of biotechnology, and specifically relates to an AAV mutant that efficiently infects supporting cells.

### Background

The cochlea of the inner ear is our peripheral sound perception organ. The auditory cells of the cochlea play a very important role in our perception of peripheral sounds. They convert external sound waves into electrophysiological signals, which are then gradually transmitted to the auditory center of the brain through the spiral ganglion cells of the inner ear.

Congenital hereditary or acquired hair cell death caused by various traumas can cause different degrees of hearing damage, and even lifelong deafness. According to a survey by the World Health Organization (WHO), 0.3% of newborns, 5% of people before the age of 45 and 50% of people over 70 have varying degrees of hearing impairment. Hearing impairment not only affects the hearing itself, but can also cause different degrees of social barriers.

Among them, hereditary deafness is caused by mutations in certain genes in the inner ear. At the same time, the low efficiency of gene transfer in inner ear cells not only affects the research of inner ear gene function, but also hinders gene therapy for hereditary deafness.

There are more than 100 genes known to cause deafness. The highest proportion is the GJB2 mutation, which accounts for about 50% of hereditary deafness; the second is the SLC26A4 gene mutation, which accounts for about 15% of hereditary deafness. Both genes are expressed in supporting cells. Myo15A and OTOF genes are expressed in hair cells, which account for 5-8% of hereditary deafness, respectively.

Therefore, it is very important to screen out vectors that can efficiently transduce into inner ear cells, especially inner ear supporting cells.

Adeno-associated virus (AAV) has broad application prospects in the field of human gene therapy. Because of its long-term gene expression ability and non-pathogenicity, it has been widely used in various studies in the liver, muscle, heart, brain, eye, kidney and other tissues.

However, there are few AAV vectors that can efficiently infect inner ear cells, especially inner ear supporting cells.

Therefore, there is an urgent need in the art to develop an easily available method that can efficiently infect supporting cells.

### Summary of the invention

The purpose of the present invention is to provide a method that is easy to be obtained and can efficiently infect supporting cells. Specifically, the purpose of the present invention is to provide an AAV mutant capable of efficiently infecting inner ear supporting cells.

Another object of the present invention is to provide a pharmaceutical composition comprising the AAV mutant provided by the present invention.

Another object of the present invention is to provide a use of the AAV mutant of the present invention for the preparation of a preparation or pharmaceutical composition for the treatment of hearing disorders.

In a first aspect of the present invention, it provides a viral capsid protein mutant, the viral capsid protein mutant is a non-natural protein, and relative to the wild-type viral capsid protein, the viral capsid protein mutant has one or more amino acid mutations selected from the group consisting of:
Serine (S) at position 670 is mutated to alanine (A), threonine (T) at position 251 is mutated to alanine (A), and lysine (K) at position 534 is mutated to arginine (R);
wherein, positions 670, 251 and 534 correspond to positions 670, 251 and 534 of the sequence as shown in SEQ ID NO: 1.

In another preferred embodiment, the viral capsid protein mutant has the activity of promoting the AAV vector to infect inner ear supporting cells.

In another preferred embodiment, the AAV vector is an AAV-DJ vector.

In another preferred embodiment, the amino acid sequence of the wild-type viral capsid protein is shown in SEQ ID NO:1.

In another preferred embodiment, except for the mutation (such as amino acid 670, 251 or 534), the remaining amino acid sequence of the viral capsid protein mutant is the same or substantially the same as the sequence as shown in SEQ ID NO: 1.

In another preferred embodiment, the substantially the same is at most 50 (preferably 1-20, more preferably 1-10, more preferably 1-5) amino acids are not the same, wherein the not the same includes amino acids substitution, deletion or addition, and the viral capsid protein mutant still has the activity of promoting the AAV vector to infect the inner ear supporting cells.

In another preferred embodiment, the serine (S) at position 670 of the viral capsid protein mutant is mutated to alanine (A), and has an amino acid sequence selected from the group consisting of:
(a) the amino acid sequence as shown in SEQ ID NO: 2;
(b) an amino acid sequence that has at least 80%, preferably at least 85% or 90%, more preferably at least 95%, most preferably at least 98% homology with the amino acid sequence as shown in SEQ ID NO: 2;
wherein, position 670 corresponds to the position 670 of the sequence as shown in SEQ ID NO: 1.

In another preferred embodiment, the threonine (T) at position 251 of the viral capsid protein mutant is mutated to alanine (A), and has an amino acid sequence selected from the group consisting of:
(a) the amino acid sequence as shown in SEQ ID NO: 3;
(b) an amino acid sequence that has at least 80%, preferably at least 85% or 90%, more preferably at least 95%, most preferably at least 98% homology with the amino acid sequence as shown in SEQ ID NO: 3;
wherein, position 251 corresponds to the position 251 of the sequence as shown in SEQ ID NO: 1.

In another preferred embodiment, the lysine (K) at position 534 of the viral capsid protein mutant is mutated to arginine (R) and has an amino acid sequence selected from the group consisting of:
(a) the amino acid sequence as shown in SEQ ID NO: 4;
(b) an amino acid sequence that has at least 80%, preferably at least 85% or 90%, more preferably at least 95%, most preferably at least 98% homology with the amino acid sequence as shown in SEQ ID NO: 4;
wherein, position 534 corresponds to the position 534 of the sequence as shown in SEQ ID NO: 1.

In a second aspect of the present invention, it provides a gene expression vector for the treatment of the hearing disorders, the expression vector is an AAV mutant, wherein in the AAV mutant, an expression cassette for a therapeutic gene for the treatment of the hearing disorders is inserted or carried;

in addition, the AAV mutant contains a gene sequence encoding the viral capsid protein mutant as described in the first aspect of the present invention.

In another preferred embodiment, the AAV mutant is an AAV-DJ mutant.

In another preferred embodiment, the AAV mutant contains a gene sequence encoding the amino acid sequence as shown in SEQ ID NO: 2, 3 or 4.

In another preferred embodiment, the AAV mutant contains a gene sequence encoding the amino acid sequence as shown in SEQ ID NO: 2.

In another preferred embodiment, the therapeutic gene includes: a hearing-related gene expressed in a normal individual (i.e., a wild-type hearing-related gene), or a related gene for gene editing.

In another preferred embodiment, the related gene for gene editing includes: the coding gene of the viral capsid protein mutant and the guide RNA (sgRNA) targeting a specific site.

In another preferred embodiment, the hearing-related gene is a gene expressed in inner ear supporting cells.

In another preferred embodiment, the gene expressed in the inner ear supporting cells is selected from the group consisting of: GJB2, SCL26A4, GJB3, Brn4, etc., and a combination thereof.

In a third aspect of the present invention, it provides a pharmaceutical composition comprising:
(i) the gene expression vector according to the second aspect of the present invention;
(ii) a pharmaceutically acceptable carrier.

In another preferred embodiment, the component (i) accounts for 0.1-99.9 wt% of the total weight of the pharmaceutical composition, preferably 10-99.9 wt%, more preferably 70-99 wt%.

In another preferred embodiment, the pharmaceutical composition is in a liquid dosage form.

In another preferred embodiment, the dosage form of the pharmaceutical composition is an injection.

In another preferred embodiment, the pharmaceutical composition is an injection for intracochlear injection.

In another preferred embodiment, the carrier is an injection carrier, preferably, the carrier is one or more carriers selected from the group consisting of physiological saline, glucose saline, and a combination thereof.

In another preferred embodiment, the pharmaceutical composition can be used alone or in combination in the treatment of the hearing disorders.

In another preferred embodiment, the used in combination includes: used in combination with other drugs for the treatment of the hearing disorders.

In another preferred embodiment, the other drugs for the treatment of the hearing disorders include: anti-infective antibiotics, neurotrophic factor drugs, ion channel modulators, vitamin pharmaceuticals, etc., or a combination thereof.

In a fourth aspect of the present invention, it provides a use of the gene expression vector as described in the second aspect of the present invention for the preparation of a preparation or pharmaceutical composition for the treatment of the hearing disorders.

In another preferred embodiment, the preparation or pharmaceutical composition is used to treat patients with the hearing disorders caused by gene mutations in inner ear supporting cells.

In another preferred embodiment, the hearing disorders is selected from the group consisting of hereditary deafness, non-hereditary deafness, and a combination thereof.

In another preferred embodiment, the hereditary deafness includes deafness caused by factors selected from the group consisting of gene mutation, gene deletion, and a combination thereof.

In another preferred embodiment, the non-hereditary deafness includes deafness caused by factors selected from the group consisting of: drug use, trauma, infection, aging, and a combination thereof.

In a fifth aspect of the present invention, it provides a method for treating hearing disorders by administering the gene expression vector as described in the second aspect of the present invention to a subject in need.

In another preferred embodiment, the method of administration is intracochlear injection.

In another preferred embodiment, the cause of the hearing disorders is a mutation of a hearing-related gene expressed in the supporting cells of the inner ear.

In another preferred embodiment, the dosage of the gene expression vector is 1×l0¹¹-5×10¹²vg, preferably 5×10¹¹-4×10¹²vg, more preferably 1×10¹²-3×10¹²vg.

In a sixth aspect of the present invention, it provides a method for preparing the gene expression vector according to the second aspect of the present invention, ligating the expression cassette of the therapeutic gene for the treatment of hearing disorders into the AAV mutant, thereby obtaining the gene expression vector according to the second aspect of the present invention.

In a seventh aspect of the present invention, it provides a method for transfecting a hearing-related cell in vitro, comprising the steps of: transfecting the hearing-related cell with an AAV mutant;
wherein, the AAV mutant contains a gene sequence encoding the viral capsid protein mutant as described in the first aspect of the present invention;
in addition, the hearing-related cell is an inner ear supporting cell.

In an eighth aspect of the present invention, it provides an isolated polynucleotide, which encodes the viral capsid protein mutant as described in the first aspect of the present invention.

In another preferred embodiment, the polynucleotide is selected from the group consisting of: DNA sequence, RNA sequence, and a combination thereof.

In a ninth aspect of the present invention, it provides a vector, which contains the polynucleotide according to the eighth aspect of the present invention.

In another preferred embodiment, the vector includes an expression vector, a shuttle vector, and an integration vector.

In a tenth aspect of the present invention, it provides a host cell, which contains the vector as described in the ninth aspect of the present invention, or its genome integrates the polynucleotide as described in the eighth aspect of the present invention.

In another preferred embodiment, the host is a prokaryotic cell or a eukaryotic cell.

In another preferred embodiment, the prokaryotic cell includes: Escherichia coli.

In another preferred embodiment, the eukaryotic cell is selected from the group consisting of a yeast cell, plant cell, mammalian cell, human cell (such as HEK293T cell), and a combination thereof.

It should be understood that, within the scope of the present invention, each technical feature of the present invention described above and in the following (as examples) may be combined with each other to form a new or preferred technical solution, which is not listed here due to space limitations.

### Description of Figure

Figure 1 shows the design of screening experiment of AAV-DJ and its mutants on supporting cell infection in mice.

Among them, the viruses of AAV-DJ and its mutants (10 species) are packaged separately, and then injected into the cochlea of P1 ICR mice. After 2-3 weeks, the samples are taken to perform fluorescence observation and phenotypic analysis of the infection of supporting cells.

Figure 2 shows analysis of the efficiency of AAV-DJ and its mutants infecting HEK293T cells in vitro.

Among them, AAV-DJ and its mutant viruses are infected with HEK293T cells in vitro, and the expression of tdTomato is observed by fluorescence microscope after 48 hours. The titer and dosage of AAV-DJ and its mutant virus are shown in the figure.

Figure 3 shows the infection results of AAV-DJ and its mutants on the supporting cells in the apical part of the mouse cochlea.

After injection of AAV-DJ and its mutant virus, the representative fluorescence image of the supporting cells at the top is shown in the figure. P1 ICR mice are collected 3 weeks after virus injection. Each mouse is injected with 4×10⁹ vg AAV virus.

Figure 4 shows the infection results of AAV-DJ and its mutants on supporting cells in the middle part of the mouse cochlea.

After injection of AAV-DJ and its mutant virus, the representative fluorescence image of the supporting cells in the middle part is shown in the figure. P1 ICR mice are collected 3 weeks after virus injection. Each mouse is injected with 4×10⁹ vg AAV virus.

Figure 5 shows the infection results of AAV-DJ and its mutants on supporting cells in the basal part of the mouse cochlea.

After injection of AAV-DJ and its mutant virus, the representative fluorescence image of the supporting cells in the basal part is shown in the figure. P1 ICR mice are collected 3 weeks after virus injection. Each mouse is injected with 4×10⁹ vg AAV virus.

Figure 6 shows the statistical analysis results of infection of AAV-DJ and its mutants on mouse cochlear supporting cells.

(A-C) the infection efficiency is explained by counting the proportion of mCherry+ cells in the top (A), middle (B) and basal (C) supporting cells in a random 100 micron field of view. AAV-DJ and its mutant virus are collected 3 weeks after injection of P1 ICR mouse cochlea. Each group of mice is injected with 4×10⁹ vg AAV virus. The results are obtained from at least 3 mice and are expressed as mean ± standard deviation. ^{∗}P<0.05, ^{∗∗∗}P<0.001, unpaired T test.

### Detailed Description

After extensive and in-depth research, after a large number of screenings, the inventors have developed an AAV mutant capable of efficiently infecting supporting cells of the inner ear for the first time. Specifically, the inventors have modified the cap sequence of AAV-DJ; among them, the specific tyrosine, serine, threonine, or lysine sites in AAV-DJ are mutated to study whether the mutation site can further improve the infection efficiency in supporting cells, and screen a large number of different mutations. In the experiment, the mutant viruses expressing tdTomato are packaged separately and injected into the cochlea of mice. Three weeks after injection, immunofluorescence analysis is performed on the cells of cochlea (Apical, Middle, Basal). Experimental results show that in supporting cells, the AAV-DJ mutant (S670A) can significantly improve the infection efficiency of supporting cells. The present invention has been completed on this basis.

### Viral Capsid Protein Mutant

In the present invention, a viral capsid protein mutant is provided, the viral capsid protein mutant is a non-natural protein, and the viral capsid protein mutant has one or more amino acid mutations selected from the group consisting of in the wild-type viral capsid protein:
Serine (S) at position 670 is mutated to alanine (A), threonine (T) at position 251 is mutated to alanine (A), and lysine (K) at position 534 is mutated to arginine (R);
wherein, the positions 670, 251 and 534 correspond to the positions 670, 251 and 534 of the sequence as shown in SEQ ID NO:1.

In one embodiment of the present invention, the amino acid sequence of the wild-type viral capsid protein is shown in SEQ ID NO:1.

MAADGYLPDWLEDTLSEGIRQWWKLKPGPPPPKPAERHKDDSRGLVLPG
YKYLGPFNGLDKGEPVNEADAAALEHDKAYDRQLDSGDNPYLKYNHADAEFQ
ERLKEDTSFGGNLGRAVFQAKKRLLEPLGLVEEAAKTAPGKKRPVEHSPVEPDS
SSGTGKAGQQPARKRLNFGQTGDADSVPDPQPIGEPPAAPSGVGSLTMAAGGG
APMADNNEGADGVGNSSGNWHCDSTWMGDRVITTSTRTWALPTYNNHLYKQ
ISNSTSGGSSNDNAYFGYSTPWGYFDFNRFHCHFSPRDWQRLINNNWGFRPKRL
SFKLFNIQVKEVTQNEGTKTIANNLTSTIQVFTDSEYQLPYVLGSAHQGCLPPFP
ADVFMIPQYGYLTLNNGSQAVGRSSFYCLEYFPSQMLRTGNNFQFTYTFEDVPF
HSSYAHSQSLDRLMNPLIDQYLYYLSRTQTTGGTTNTQTLGFSQGGPNTMANQ
AKNWLPGPCYRQQRVSKTSADNNNSEYSWTGATKYHLNGRDSLVNPGPAMAS
HKDDEEKFFPQSGVLIFGKQGSEKTNVDIEKVMITDEEEIRTTNPVATEQYGSVS
TNLQRGNRQAATADVNTQGVLPGMVWQDRDVYLQGPIWAKIPHTDGHFHPSP
LMGGFGLKHPPPQILIKNTPVPADPPTTFNQSKLNSFITQYSTGQVSVEIEWELQK
ENSKRWNPEIQYTSNYYKSTSVDFAVNTEGVYSEPRPIGTRYLTRNL* （SEQ ID
NO: 1）

In a preferred embodiment of the present invention, the amino acid sequence of the viral capsid protein mutant is shown in SEQ ID NO: 2, wherein the S670A mutation occurs corresponding to the wild-type viral capsid protein sequence.

MAADGYLPDWLEDTLSEGIRQWWKLKPGPPPPKPAERHKDDSRGLVLPG
YKYLGPFNGLDKGEPVNEADAAALEHDKAYDRQLDSGDNPYLKYNHADAEFQ
ERLKEDTSFGGNLGRAVFQAKKRLLEPLGLVEEAAKTAPGKKRPVEHSPVEPDS
SSGTGKAGQQPARKRLNFGQTGDADSVPDPQPIGEPPAAPSGVGSLTMAAGGG
APMADNNEGADGVGNSSGNWHCDSTWMGDRVITTSTRTWALPTYNNHLYKQ
ISNSTSGGSSNDNAYFGYSTPWGYFDFNRFHCHFSPRDWQRLINNNWGFRPKRL
SFKLFNIQVKEVTQNEGTKTIANNLTSTIQVFTDSEYQLPYVLGSAHQGCLPPFP
ADVFMIPQYGYLTLNNGSQAVGRSSFYCLEYFPSQMLRTGNNFQFTYTFEDVPF
HSSYAHSQSLDRLMNPLIDQYLYYLSRTQTTGGTTNTQTLGFSQGGPNTMANQ
AKNWLPGPCYRQQRVSKTSADNNNSEYSWTGATKYHLNGRDSLVNPGPAMAS
HKDDEEKFFPQSGVLIFGKQGSEKTNVDIEKVMITDEEEIRTTNPVATEQYGSVS

TNLQRGNRQAATADVNTQGVLPGMVWQDRDVYLQGPIWAKIPHTDGHFHPSP
LMGGFGLKHPPPQILIKNTPVPADPPTTFNQSKLNAFITQYSTGQVSVEIEWELQ
KENSKRWNPEIQYTSNYYKSTSVDFAVNTEGVYSEPRPIGTRYLTRNL* （SEQ
ID NO: 2）

In a preferred embodiment of the present invention, the amino acid sequence of the viral capsid protein mutant is shown in SEQ ID NO: 3, wherein the T251A mutation occurs corresponding to the wild-type viral capsid protein sequence.

MAADGYLPDWLEDTLSEGIRQWWKLKPGPPPPKPAERHKDDSRGLVLPG
YKYLGPFNGLDKGEPVNEADAAALEHDKAYDRQLDSGDNPYLKYNHADAEFQ
ERLKEDTSFGGNLGRAVFQAKKRLLEPLGLVEEAAKTAPGKKRPVEHSPVEPDS
SSGTGKAGQQPARKRLNFGQTGDADSVPDPQPIGEPPAAPSGVGSLTMAAGGG
APMADNNEGADGVGNSSGNWHCDSTWMGDRVITTSTRTWALPAYNNHLYKQ
ISNSTSGGSSNDNAYFGYSTPWGYFDFNRFHCHFSPRDWQRLINNNWGFRPKRL
SFKLFNIQVKEVTQNEGTKTIANNLTSTIQVFTDSEYQLPYVLGSAHQGCLPPFP
ADVFMIPQYGYLTLNNGSQAVGRSSFYCLEYFPSQMLRTGNNFQFTYTFEDVPF
HSSYAHSQSLDRLMNPLIDQYLYYLSRTQTTGGTTNTQTLGFSQGGPNTMANQ
AKNWLPGPCYRQQRVSKTSADNNNSEYSWTGATKYHLNGRDSLVNPGPAMAS
HKDDEEKFFPQSGVLIFGKQGSEKTNVDIEKVMITDEEEIRTTNPVATEQYGSVS
TNLQRGNRQAATADVNTQGVLPGMVWQDRDVYLQGPIWAKIPHTDGHFHPSP
LMGGFGLKHPPPQILIKNTPVPADPPTTFNQSKLNSFITQYSTGQVSVEIEWELQK
ENSKRWNPEIQYTSNYYKSTSVDFAVNTEGVYSEPRPIGTRYLTRNL* （SEQ ID
NO: 3）

In a preferred embodiment of the present invention, the amino acid sequence of the viral capsid protein mutant is shown in SEQ ID NO: 4, wherein a K534R mutation occurs corresponding to the wild-type viral capsid protein sequence.

MAADGYLPDWLEDTLSEGIRQWWKLKPGPPPPKPAERHKDDSRGLVLPG
YKYLGPFNGLDKGEPVNEADAAALEHDKAYDRQLDSGDNPYLKYNHADAEFQ
ERLKEDTSFGGNLGRAVFQAKKRLLEPLGLVEEAAKTAPGKKRPVEHSPVEPDS
SSGTGKAGQQPARKRLNFGQTGDADSVPDPQPIGEPPAAPSGVGSLTMAAGGG
APMADNNEGADGVGNSSGNWHCDSTWMGDRVITTSTRTWALPTYNNHLYKQ

ISNSTSGGSSNDNAYFGYSTPWGYFDFNRFHCHFSPRDWQRLINNNWGFRPKRL
SFKLFNIQVKEVTQNEGTKTIANNLTSTIQVFTDSEYQLPYVLGSAHQGCLPPFP
ADVFMIPQYGYLTLNNGSQAVGRSSFYCLEYFPSQMLRTGNNFQFTYTFEDVPF
HSSYAHSQSLDRLMNPLIDQYLYYLSRTQTTGGTTNTQTLGFSQGGPNTMANQ
AKNWLPGPCYRQQRVSKTSADNNNSEYSWTGATKYHLNGRDSLVNPGPAMAS
HKDDEERFFPQSGVLIFGKQGSEKTNVDIEKVMITDEEEIRTTNPVATEQYGSVS
TNLQRGNRQAATADVNTQGVLPGMVWQDRDVYLQGPIWAKIPHTDGHFHPSP
LMGGFGLKHPPPQILIKNTPVPADPPTTFNQSKLNSFITQYSTGQVSVEIEWELQK
ENSKRWNPEIQYTSNYYKSTSVDFAVNTEGVYSEPRPIGTRYLTRNL* （SEQ ID
NO: 4）

The present invention also includes polypeptides or proteins with the same or similar functions that have 50% or more (preferably 60% or more, 70% or more, 80% or more, more preferably 90% or more, more preferably 95% or more, most preferably 98% or more, such as 99%) homology with the sequence as shown in SEQ ID NO: 2, 3 or 4 of the present invention.

The "same or similar function" mainly refers to: "promoting the activity of the AAV vector to infect the inner ear supporting cells". Preferably, the AAV vector is an AAV-DJ vector.

It should be understood that the amino acid numbering in the viral capsid protein mutants of the present invention is based on SEQ ID NO: 2, 3 or 4. When the homology of a specific viral capsid protein mutant with the sequence as shown in SEQ ID NO: 2, 3 or 4 reaches 80% or more, the amino acid numbering of the viral capsid protein mutant may be misplaced relative to the amino acid numbering of SEQ ID NO: 2, 3, or 4, such as shifting positions 1-5 to the N-terminus or C-terminus of the amino acid, and using conventional sequence alignment techniques in the art, those skilled in the art can generally understand that such a misalignment is within a reasonable range, and should not be due to misplacement of amino acid numbering, mutants with the same or similar catalytic activity for producing viral capsid protein mutants that have homology to 80% (such as 90%, 95%, 98%) are not within the scope of the viral capsid protein mutants of the present invention.

The viral capsid protein mutant of the present invention is a synthetic protein or a recombinant protein, that is, it can be a chemically synthesized product, or produced from a prokaryotic or eukaryotic host (for example, bacteria, yeast, plants) using recombinant technology. Depending on the host used in the recombinant production protocol, the viral capsid protein mutant of the present invention may be glycosylated or non-glycosylated. The viral capsid protein mutants of the present invention may also include or not include the initial methionine residue.

The present invention also includes fragments, derivatives and analogs of the viral capsid protein mutants. As used herein, the terms "fragment", "derivative" and "analog" refer to a protein that substantially retains the same biological function or activity as the viral capsid protein mutant.

The viral capsid protein mutant fragment, derivative or analogue of the present invention may be (i) a viral capsid protein mutant with one or more conservative or non-conservative amino acid residues (preferably conservative amino acid residues) substituted, and such substituted amino acid residues may or may not be encoded by the genetic code, or (ii) a viral capsid protein mutant with substitution groups in one or more amino acid residues, or (iii) a viral capsid protein mutant formed by fusion of a mature viral capsid protein mutant with another compound (such as a compound that extends the half-life of the viral capsid protein mutant, such as polyethylene glycol), or (iv) an additional amino acid sequence is fused to this viral capsid protein mutant sequence to form a viral capsid protein mutant (such as the leader sequence or secretory sequence or the sequence or the proprotein sequence used to purify the viral capsid protein mutant, or the fusion protein formed with the antigen IgG fragment). According to the teachings herein, these fragments, derivatives and analogs fall within the scope of those skilled in the art. In the present invention, conservatively substituted amino acids are preferably generated by amino acid substitutions according to Table I.

**Table I**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala;phe | Leu |
| Leu (L) | Ile; Val; Met; Ala;phe | Ile |
| Lys (K) | Arg; Gln; Asn | Arg |
| Met (M) | Leu;phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr;phe | Tyr |
| Tyr (Y) | Trp;phe; Thr; Ser | Phe |
| Val (V) | Ile; Leu; Met;phe; Ala | Leu |

In addition, the capsid protein mutant of the virus of the present invention can also be modified. Modified (usually unchanged primary structure) forms include: chemically derived forms of viral capsid protein mutants in vivo or in vitro, such as acetylation or carboxylation. Modifications also include glycosylation, such as those produced by glycosylation modification during the synthesis and processing or during further processing steps of viral capsid protein mutants. This modification can be accomplished by exposing the viral capsid protein mutant to an enzyme that performs glycosylation (such as mammalian glycosylase or deglycosylase). Modified forms also include sequences with phosphorylated amino acid residues (such as phosphotyrosine, phosphoserine, phosphothreonine). It also includes mutants of the viral capsid protein that have been modified to improve their resistance to proteolysis or to optimize their solubility.

The term "polynucleotide encoding a viral capsid protein mutant" may include a polynucleotide encoding the viral capsid protein mutant of the present invention, or may also be a polynucleotide that also includes additional coding and/or non-coding sequences.

The present invention also relates to variants of the above-mentioned polynucleotides, which encode fragments, analogs and derivatives of polypeptides or viral capsid protein mutants having the same amino acid sequence as the present invention. These nucleotide variants include substitution variants, deletion variants and insertion variants. As known in the art, an allelic variant is an alternative form of a polynucleotide. It may be a substitution, deletion or insertion of one or more nucleotides, but will not substantially change the function of the encoded viral capsid protein mutant.

The present invention also relates to polynucleotides that hybridize with the aforementioned sequences and have at least 50%, preferably at least 70%, and more preferably at least 80% identity between the two sequences. The present invention particularly relates to polynucleotides that can hybridize with the polynucleotide of the present invention under strict conditions (or stringent conditions). In the present invention, " strict conditions" refer to: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0.1% Ficoll, 42°C, etc.; or (3) Hybridization occurs only when the identity between the two sequences is at least 90% or more, and more preferably 95% or more.

The viral capsid protein mutants and polynucleotides of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and using a commercially available cDNA library or a cDNA library prepared according to a conventional method known to those skilled in the art as a template, amplifying the relevant sequence. When the sequence is long, it is often necessary to perform two or more PCR amplifications, and then the amplified fragments are spliced together in the correct order.

Once the relevant sequence is obtained, the recombination method can be used to obtain the relevant sequence in large quantities. This is usually done by cloning it into a vector, then transferring it into a cell, and then isolating the relevant sequence from the proliferated host cell by conventional methods.

In addition, artificial synthesis methods can also be used to synthesize related sequences, especially when the fragment length is short. Usually, by first synthesizing multiple small fragments, and then ligating to obtain fragments with very long sequences.

At present, the DNA sequence encoding the protein (or fragment or derivative thereof) of the present invention can be obtained completely through chemical synthesis. The DNA sequence can then be introduced into various existing DNA molecules (or such as vectors) and cells known in the art. In addition, mutations can also be introduced into the protein sequence of the present invention through chemical synthesis.

The method of using PCR technology to amplify DNA/RNA is preferably used to obtain the polynucleotide of the present invention. Especially when it is difficult to obtain full-length cDNA from the library, the RACE method (RACE-cDNA end rapid amplification method) can be preferably used. The primers used for PCR can be appropriately selected according to the sequence information of the present invention disclosed herein and can be synthesized by conventional methods. The amplified DNA/RNA fragments can be separated and purified by conventional methods such as gel electrophoresis.

### Gene expression vector of the present invention

As used herein, the terms "gene expression vector" and "AAV mutant" can be used interchangeably and refer to the AAV mutant of the present invention, wherein in the AAV mutant, an expression cassette for a therapeutic gene for the treatment of hearing disorders is inserted or carried; and, the AAV mutant contains a gene sequence encoding the viral capsid protein mutant as described in the first aspect of the present invention.

In a preferred embodiment, the AAV mutant is an AAV-DJ mutant.

Preferably, the therapeutic gene includes: a hearing-related gene expressed in normal individuals (i.e., a wild-type hearing-related gene), or a related gene for gene editing.

In a preferred embodiment, the related gene used for gene editing includes: the coding gene of the viral capsid protein mutant and the guide RNA (sgRNA) targeting a specific site.

In another preferred embodiment, the hearing-related gene is a gene expressed in inner ear supporting cells.

In one embodiment of the present invention, the gene expressed in the inner ear supporting cells is selected from the group consisting of: GJB2, SCL26A4, GJB3, Brn4, etc., and a combination thereof.

In a preferred embodiment, the AAV mutant of the present invention contains a gene sequence encoding the amino acid sequence as shown in SEQ ID NO: 2.

### Pharmaceutical composition and method of administration

In the present invention, it further provides a pharmaceutical composition, which contains (i) a safe and effective amount of the gene expression vector according to the first aspect of the present invention; (ii) a pharmaceutically acceptable carrier.

As used herein, the term "including" includes "containing", "consisting essentially of' and "consisting of".

As used herein, the term "consisting essentially of' means that in addition to the effective active ingredient or auxiliary ingredient, the pharmaceutical composition may also contain a small amount of minor ingredients and/or impurities that do not affect the active ingredient.

As used herein, "pharmaceutically acceptable" ingredients are substances that are suitable for humans and/or mammals without excessive side effects (such as toxicity, irritation, and allergic reactions), that is, substances that have a reasonable benefit/risk ratio.

The term "pharmaceutically acceptable carrier" refers to a carrier used for the administration of a therapeutic agent, and includes various excipients and diluents. The term refers to such pharmaceutical carriers: they are not essential active ingredients themselves, and there is no excessive toxicity after administration. Suitable carriers are well known to those of ordinary skill in the art.

The pharmaceutically acceptable carriers of the present invention include (but are not limited to): water, saline, liposomes, lipids, proteins, protein-antibody conjugates, peptides, cellulose, nanogels, or a combination thereof. The choice of carrier should match the mode of administration, which are well known to those of ordinary skill in the art.

The pharmaceutical composition of the present invention contains a safe and effective amount of the active ingredient of the present invention and a pharmaceutically acceptable carrier. Such carriers include (but are not limited to): saline, buffer, glucose, water, glycerol, ethanol, and a combination thereof. Generally, the pharmaceutical preparation should match the mode of administration. The dosage form of the pharmaceutical composition of the present invention is injection, oral preparation (tablet, capsule, oral liquid), transdermal agent, and sustained-release agent. For example, it can be prepared by conventional methods with physiological saline or an aqueous solution containing glucose and other adjuvants. The pharmaceutical composition should be manufactured under aseptic conditions.

In a preferred embodiment, the pharmaceutical composition is in a liquid dosage form.

In a more preferred embodiment, the dosage form of the pharmaceutical composition is an injection. Preferably, the pharmaceutical composition of the present invention is an injection form for intracochlear injection.

In one embodiment of the present invention, the carrier is an injection carrier. Preferably, the carrier is one or more carriers selected from the group consisting of physiological saline, dextrose saline, and a combination thereof.

In one embodiment of the present invention, the pharmaceutical composition can be used alone or in combination in the treatment of hearing disorders.

In the present invention, the used in combination includes: used in combination with other drugs for the treatment of hearing disorders.

In a more preferred embodiment, the other drugs for the treatment of hearing disorders include: anti-infective antibiotic drugs, neurotrophic factor drugs, ion channel modulator drugs, vitamin drugs, etc., or a combination thereof.

As used herein, the term "effective amount" or "effective dose" refers to an amount that can produce function or activity on humans and/or animals and can be accepted by humans and/or animals.

The effective amount of the active ingredient of the present invention can vary with the mode of administration and the severity of the disease to be treated. The selection of the preferred effective amount can be determined by a person of ordinary skill in the art according to various factors (for example, through clinical trials). The factors include, but are not limited to: the pharmacokinetic parameters of the active ingredients such as bioavailability, metabolism, half-life, etc.; the severity of the disease to be treated by the patient, the patient's weight, the patient's immune status, and route of administration, etc.. For example, due to the urgent request of the treatment condition, several divided doses can be given every day, or the dose can be reduced proportionally.

### The main advantages of the present invention include:

1) High efficiency: AAV-DJ mutant has a high infection efficiency on inner ear supporting cells.
2) Ease of production: The AAV-DJ mutant vector of the present invention has a high virus detection rate and high stability, and it is easy to obtain high-titer and high-quality AAV during the production process.
3) Good safety: AAV is a vector approved by the FDA for clinical treatment, and the AAV-DJ mutant vector of the present invention has no damage to inner ear tissue.
4) High targeting: Compared with small molecule drugs, the AAV-DJ mutant vector of the present invention has the characteristics of tissue and cell-specific infection, and can target specific cell types.

The present invention will be further explained below in conjunction with specific embodiments. It should be understood that these embodiments are only used to illustrate the present invention and not to limit the scope of the present invention. The experimental methods without specific conditions in the following examples usually follow the conventional conditions, such as the conditions described in Sambrook et al., Molecular Cloning: Laboratory Manual (New York: Cold Spring Harbor Laboratory Press, 1989), or according to manufacturing The conditions suggested by the manufacturer. Unless otherwise specified, percentages and parts are weight percentages and parts by weight.

### Experimental materials and methods

### Mouse

ICR mice (P1) were used for AAV virus injection. The use and care of animals were completed under the guidance of the animal ethics committee.

### Cell culture and infection

HEK293T cells used 10% FBS medium, the ingredients were Dulbecco's modified Eagle medium (DMEM) (Gibco, 11965-02), 10% fetal bovine serum (FBS) (Gibco), 2mM glutamine (Gibco), 1% penicillin/streptomycin (Thermo Fisher Scientific) and 0.1 mM non-essential amino acids (Gibco). All cells were cultured under 5% CO₂, 37°C.

48 hours after HEK293T cells were infected with AAV, the expression of tdTomato was observed by fluorescence microscope.

### AAV virus packaging

The three-plasmid system was used to transfect 293T cells, and the medium was changed 4-6 hours after transfection. Collecting the supernatant and cells on the fourth day. The supernatant was precipitated with PEG overnight, centrifuged at 4200 rpm, 4°C for 30 min, and then centrifuged at 4400 rpm for 10 min, and the supernatant was discarded. Dissolved with 1×GB. The cells were repeatedly frozen and thawed three times with liquid nitrogen. The supernatant and cells were digested with benzonase and 5M NaCl for 30 minutes. After digestion, centrifuged at 3000g for 10 minutes and taking the supernatant. Density gradient ultracentrifugation, 68000rpm 18°C 1h 25min. Taking the layer, diluted with PBS and concentrated with an ultrafiltration tube.

### AAV virus injection

ICR P1 mice, there is no restriction on male and female, are randomly grouped according to different AAV serotypes, with 4 mice in each group. Under a stereomicroscope, cutting the skin 2mm behind the posterior sulcus of auricle with ophthalmological scissors, and slightly separating the subcutaneous tissue. The facial nerve, the paries posterior of the auditory vesicle, and the posterior belly of the musculus digastricus can be seen. A glass microelectrode was used to puncture the cochlear sidewall ligament and injecting 1 microliter of virus into the mouse cochlea. After the injection, the glass electrode was gently pulled out, and the incision was sewn up. Three weeks after the injection, the samples were taken to analyze the phenotype.

### Immunostaining analysis

In the immunostaining experiment, the mice were anesthetized with Pentobarbital sodium (50mg/Kg, Sigma), and peristaltic pump (Gilson) was used for cardiac perfusion with 0.9% saline and 4% paraformaldehyde, and then fixed in 4% paraformaldehyde at 4°C overnight. The next day the tissue was decalcified in 10% EDTA. After the decalcification is completed, the basement membrane is separated under a dissecting microscope and cut into three sections (top, middle and base part). The separated basement membrane was washed three times with 0.1M phosphate buffer (PB), and then incubated with primary antibody diluted with 5% NGS at 4°C overnight. The next day, the sections were washed three times with PB, and then incubated with the secondary antibody on a rotary shaker for two hours at room temperature. Finally, the sections were counter-stained with DAPI for 20 minutes, and then mounted on a glass slide with SlowFade Diamond Antifade Mountant (Life).

### Antibody

### Supporting cells:

Primary antibody: goat-anti-Sox2 (Santa Cruz Biotechnology, sc-17320)
Secondary antibody: Alexa Fluor^{®} 488 AffiniPure Donkey Anti-Goat IgG (H+L) (Jackson ImmunoResearch, 705-545-003)

### Hair cells:

Primary antibody: rabbit anti-Myosin-VI polyclonal (Proteus Biosciences, 25-6791)
Secondary antibody: Cy^{™}5 AffiniPure Donkey Anti-Rabbit IgG (H+L) (Jackson Immuno Research, 711-175-152)

### Data statistical analysis and software

The infection efficiency in supporting cells and hair cells was quantified, and the infection efficiency was explained by counting the proportion of mCherry+ cells in the supporting cells in a random 100-micron field of view. The results are obtained from at least 3 mice and are expressed as mean ± standard deviation. ^{∗}P<0.05, ^{∗∗∗}P<0.001, unpaired T test.
Snapgene: for plasmid map construction and design
Excel: Raw data processing
NIS-Elements Viewer 4.0: Experimental image processing
ImageJ: Experimental image processing
Adobe photoshop CS6: Experimental image processing
Adobe Illustrator CS4: Experimental image processing

### Example 1: AAV-DJ mutant screening experiment design

In this example, the cap sequence of AAV-DJ was genetically modified to screen out AAV vectors that can efficiently infect supporting cells.

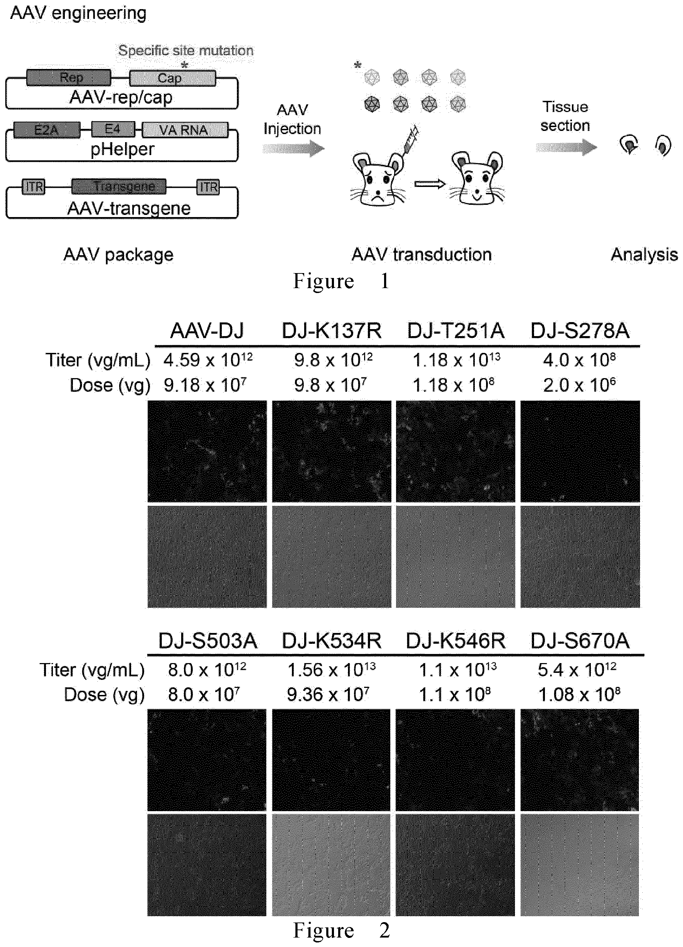

Specifically, the present inventors mutated specific tyrosine, serine, threonine or lysine sites in the cap sequence of AAV-DJ, and studied whether the mutants could further improve the infection efficiency in supporting cells (Figure 1).

Among them, 7 different AAV-DJ mutants (K137A, T251A, S278A, S503A, K534R, K546R and S670A) were designed respectively.

### Example 2: AAV-DJ mutant infects HEK293T cells in vitro

In this example, HEK293T cells were infected with AAV-DJ and its mutant viruses in vitro, respectively, and the expression of tdTomato was observed by fluorescence microscope after 48 hours.

The titer and dosage of AAV-DJ and its mutant virus are shown in the figure (Figure 2).

The results show that AAV-DJ mutants (K137A, T251A, S670A) have a higher infection efficiency than AAV-DJ in HEK293T cells in vitro.

### Example 3.

In this example, AAV-DJ and its mutant virus were respectively injected into the cochlea of P1 ICR mice. Each mouse was injected with 4×10⁹ vg AAV virus. Similarly, 3 weeks after the injection, the cochlear basement membrane of the mouse was stripped and stained, and the proportion of mCherry+ cells in the supporting cells in the top, middle and basal parts were counted, respectively.

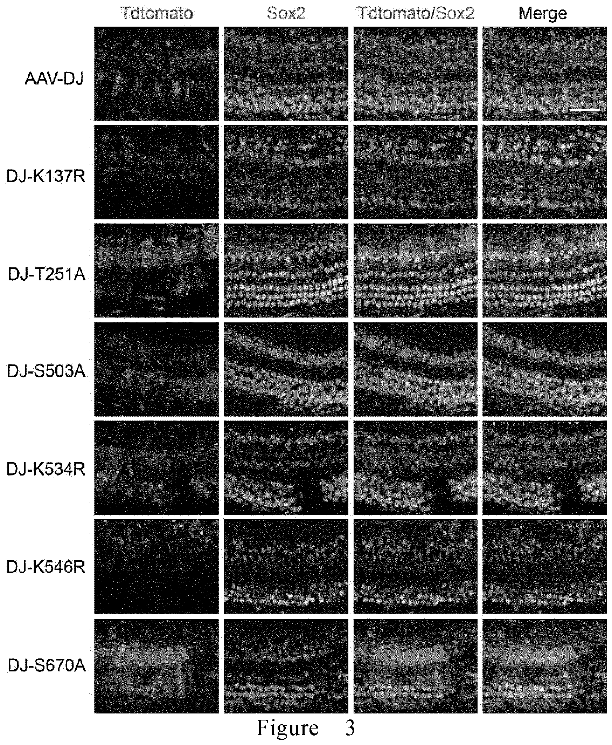

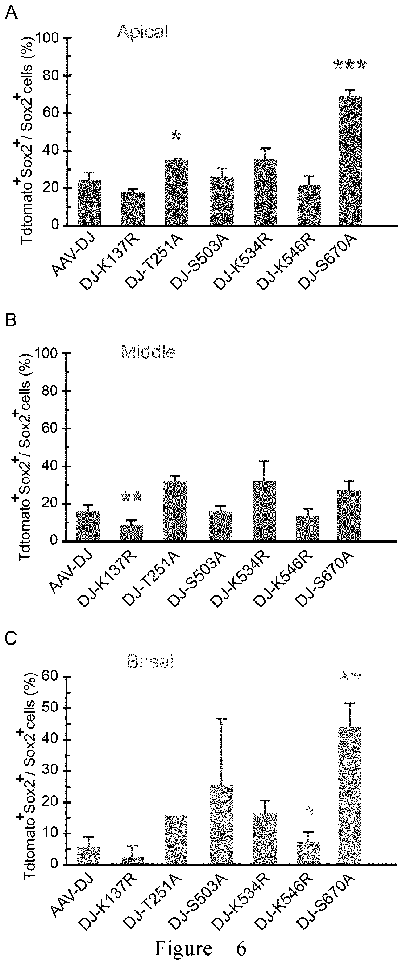

The experimental results show that in AAV-DJ mutants (T251A and S670A), 34.99±0.70% and 69.30±2.96% of the supporting cells (sox2 positive) in the top part of the AAV-DJ mutants are Tdtomato positive, which is significantly higher than the 24.64±3.79% of AAV-DJ. (Figure 3, Figure 6).

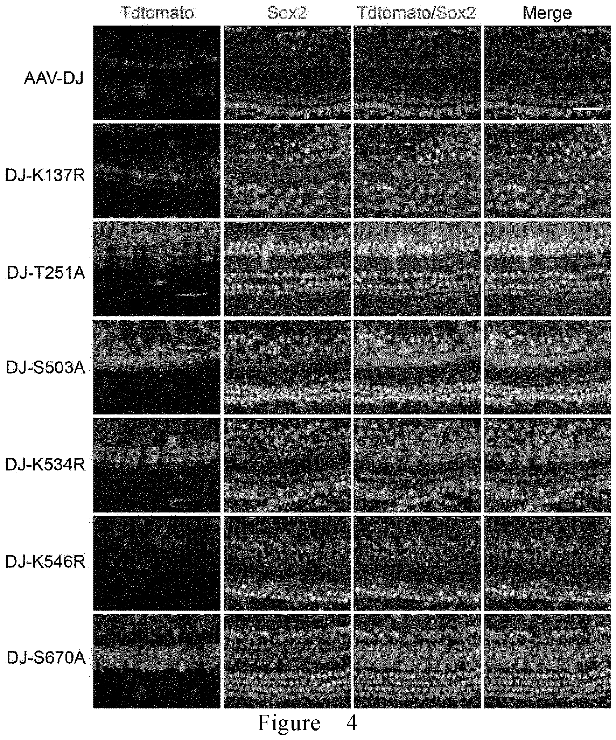

In AAV-DJ mutants (T251A, K534R and S670A), 28.01±5.01%, 31.84±10.63% and 27.38±4.61% of the supporting cells (sox2 positive) in the middle part of the AAV-DJ mutants are Tdtomato positive, which is higher than 16.1± 3.00% of AAV-DJ, but there is no significant difference (Figure 4, Figure 6).

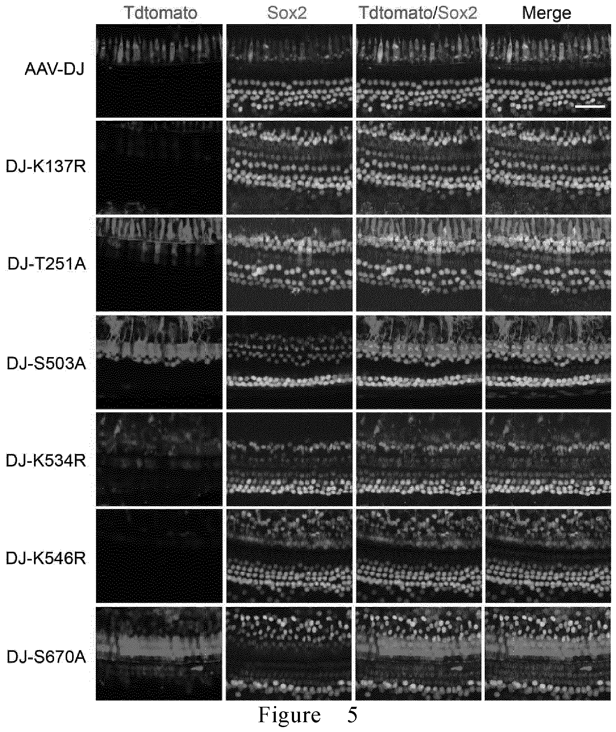

The AAV-DJ mutants (S503A, K534R and S670A) have 25.61±20.99%, 16.74±3.84% and 44.28±7.37% in the supporting cells (sox2 positive) of the basal part, respectively. 44.28±7.37% of the cells are Tdtomato positive. It is significantly higher than 5.61±3.22% of AAV-DJ (Figure 5, Figure 6).

The above experimental results show that in supporting cells, the AAV-DJ mutant (S670A) can significantly improve the infection efficiency of supporting cells, and T251A and K534R can also promote the infection of supporting cells by AAV-DJ.

### Discussion

In the present invention, in order to screen out AAV vectors that can efficiently infect supporting cells, genetic modification is performed on the cap sequence of AAV-DJ.

The ubiquitin-proteasome degradation mechanism is considered to be the main obstacle to the efficiency of AAV infection. Among them, the point mutation at the specific site of the viral capsid protein cap is the simplest and most common method, which can make the viral vector avoid intracellular phosphorylation and subsequent ubiquitination and proteasome-mediated degradation. It has been reported in the literature that specific tyrosine, serine, threonine or lysine point mutations in AAV2 and AAV8 viruses can significantly increase their infection efficiency, but they have specific liver tropism in vivo.

In a specific embodiment of the present invention, specific tyrosine, serine, threonine or lysine sites in AAV-DJ are mutated to investigate whether the mutants can further improve the infection efficiency in supporting cells.

Specifically, 7 different AAV-DJ mutants (K137A, T251A, S278A, S503A, K534R, K546R and S670A) were designed respectively. The inventors have separately packaged viruses expressing the AAV-DJ mutant of tdTomato and injected them into the cochlea of mice. Three weeks after the injection, we have performed immunofluorescence analysis on the cells of the cochlea (top, middle, and basal).

Through in vitro and in vivo experimental results, it is found that the AAV-DJ mutant (S670A) can significantly improve the infection efficiency of supporting cells. This provides great help for the study of gene function of ear supporting cells and gene therapy strategies.

In addition to point mutations at specific locations, there are other ways to modify the viral protein coats, such as inserting random sequences, random point mutations, disrupting rearrangements, and so on. All of these may further promote the improvement of the infection efficiency of mouse supporting cells, and more experiments are needed for verification.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. A viral capsid protein mutant, wherein the viral capsid protein mutant is a non-natural protein, and relative to the wild-type viral capsid protein, the viral capsid protein mutant has one or more amino acid mutations selected from the group consisting of:
Serine (S) at position 670 is mutated to alanine (A), threonine (T) at position 251 is mutated to alanine (A), and lysine (K) at position 534 is mutated to arginine (R);
wherein, positions 670, 251 and 534 correspond to positions 670, 251 and 534 of the sequence as shown in SEQ ID NO: 1.

2. The viral capsid protein mutant of claim 1, wherein the viral capsid protein mutant has the activity of promoting the AAV vector to infect inner ear supporting cells.

3. The viral capsid protein mutant of claim 1, wherein the AAV vector is an AAV-DJ vector.

4. A gene expression vector for the treatment of the hearing disorders, wherein the expression vector is an AAV mutant, wherein in the AAV mutant, an expression cassette for a therapeutic gene for the treatment of the hearing disorders is inserted or carried;
in addition, the AAV mutant contains a gene sequence encoding the viral capsid protein mutant of claim 1.

5. The gene expression vector of claim 4, wherein the AAV mutant contains a gene sequence encoding the amino acid sequence as shown in SEQ ID NO: 2, 3 or 4.

6. A pharmaceutical composition comprising:
(i) the gene expression vector of claim 4;
(ii) a pharmaceutically acceptable carrier.

7. The pharmaceutical composition of claim 6, wherein the pharmaceutical composition is an injection for intracochlear injection.

8. Use of the gene expression vector of claim 4 for the preparation of a preparation or pharmaceutical composition for the treatment of the hearing disorders.

9. A method for treating hearing disorders by administering the gene expression vector of claim 4 to a subject in need.

10. A method for preparing the gene expression vector of claim 4, wherein ligating the expression cassette of the therapeutic gene for the treatment of hearing disorders into the AAV mutant, thereby obtaining the gene expression vector of claim 4.
